# EUROPEAN PATENT SPECIFICATION

(11) **EP 1 694 381 B1**
(45) Date of publication and mention of the grant of the patent: **19.03.2008**
(21) Application number: 04820550.4
(22) Date of filing: 10.12.2004
(51) Int. Cl.: A61M 1/28

(54) **A PACKAGE FOR USE IN A PERITONEAL DIALYSIS TREATMENT AND A METHOD FOR MANUFACTURE**
PACKUNG ZUR VERWENDUNG BEI EINER PERITONEAL-DIALYSEBEHANDLUNG UND VERFAHREN ZUR HERSTELLUNG
CONDITIONNEMENT DESTINE A ETRE UTILISE DANS UN TRAITEMENT DE DIALYSE PERITONEALE ET PROCEDE DE FABRICATION

(30) Priority: 18.12.2003 SE 0303416; 19.12.2003 US 531468 P
(43) Date of publication of application: 30.08.2006
(73) Proprietor: Gambro Lundia AB, 220 10 Lund (SE)
(72) Inventor: AXELSSON, Mikael, S-244 65 Furulund (SE); NILSSON, Lars-Olof, S-234 38 Lomma (SE)
(74) Representative: Israelsson, Stefan
(86) International application number: PCT/SE2004/001843
(87) International publication number: WO 2005/058389

(56) References cited:
- EP-A1- 0 571 978
- US-A- 3 006 341
- US-A- 5 350 357
- US-A- 6 012 578
- US-B1- 6 575 954

## Description

### THE BACKGROUND OF THE INVENTION AND PRIOR ART

The present invention relates to a package for use in a peritoneal dialysis treatment and a method for manufacturing of such a package.

Such a package, which may be used in a peritoneal dialysis treatment, usually comprises a line set, a drain bag, and a solution bag, which is filled with a dialysis solution. The line set includes one or several tubular line elements and further elements as one connector connectable to a catheter, which is introduced into the abdomen of the patient by operation, one connector connectable to the drain bag and one connector connectable to the solution bag. The line set also includes flow organizers, such as clamps or at least one valve, which are used for controlling the flow in the line set. The package usually also comprises a wrapping for encasing the line set and the bags.

Such a package for use in a peritoneal dialysis treatment must be sterilized. Mostly, steam autoclave sterilization is used for this purpose. The autoclave temperature has to be at least about 120° C during a specified retention time in order to guarantee that all microorganisms are killed. Consequently, the components included in the package have to be manufactured of materials, which can withstand the autoclave temperature during the retention time. However, the tubular line elements of the line set and the bags are usually manufactured of PVC. PVC has the property that it withstands the autoclave temperature but becomes relatively soft at this temperature. Therefore, there is a risk that an area of a tubular line element, which is loaded by another line or a component in the package, is deformed during the autoclave sterilization. If an area of a tubular line element of the line set becomes permanently deformed, there is a risk that the flow of the dialysis solution through this line element will be restricted or wholly stopped.
US 6 012 578 shows a package for use in a peritoneal dialysis treatment. The package comprises two bags and a line set. These components are manufactured of polyvinyl chloride (PVC). The bags and the line set are arranged in an overpouch (wrapping). The line set is organized in the package such that several areas of the line elements of the line set are loaded by other parts of the line elements. Consequently, there is a risk that the loaded areas of the line elements are deformed during an autoclave sterilization of the package.

Normally, a patient himself may use such a package for draining fluid from the abdominal cavity and filling fresh dialysis solution to the abdominal cavity. The patient then opens the package and connects the line set, via the connector, to the catheter. The clamps are controlled such that used fluid contained in the abdominal cavity is drained through the line set to the drain bag. Thereafter, the patient adjusts the clamps such that new dialysis solution is supplied from the solution bag to the abdominal cavity via the line set.

### SUMMARY OF THE INVENTION

The object of the present invention is to provide a package for use in a peritoneal dialysis treatment, which functions in a reliable manner. A further object is to provide such a package, in which the risk that any part of the tubular line elements in the line set is deformed during an autoclave sterilizing process is substantially eliminated.

The object is achieved by the package initially defined, which comprises organizing means arranged to organize the line set such that in the package no part of the line set extends across another part of the line set. If a part of the line set extends across another part of the line set, there is always a risk that an upper part of the line set get in contact with a lower part of the line set and exerts a load thereon with a certain pressure. Since many plastic materials become relatively soft during an autoclave sterilization process, there is a risk that such a loaded part of the line set is deformed. However, by the organizing of the line set, according to the present invention, this risk may be reduced or even substantially eliminated. Consequently, the risk for a part of the line set being deformed during the autoclave sterilization is considerably reduced.

According to an embodiment of the invention, the organizing means are arranged to organize the whole line set at substantially the same level. By such an organizing, no part of the line set extends at a higher level than any other part of the line set- Consequently, no part of the line set could lie on and thus exert a load on another part of the line set. The line et takes up a very small space in a direction perpendicular to said same level. Consequently, the package may be relatively compact. The organizing means may be arranged to organize the line set such that no part of the tubular line elements is in contact with another part of the tubular line elements. The flexible line elements of a line set are often manufactured in plastic materials, which become relatively soft during the autoclave sterilization. In such a case, there is a risk for the contact areas of the line elements to adhere to each other. By the above-mentioned organizing of the line element or elements, this risk is substantially eliminated.

According to a further embodiment of the invention, the organizing means are arranged to organize the line set in a spiral-shaped state. In this case, the line set may be wound along a substantially circular path or the like. The end parts of the line set may be arranged radially outside or radially inside the wound line set. Mostly, the end parts of the line elements are provided with connectors. The space for positioning of the connectors is in these positions relatively large. The risk for a connector to be positioned such that it exerts a load on an adjacent line element is then negligible.

According to a further embodiment of the invention, at least one tubular line element of the line set is pre-shaped to extend along a desired path. In this way, the flexible line element automatically attains a desired shape in the package. Preferably, all line elements of the line set may be pre-shaped. Thereby, the whole line set tries to extend along a desired path in which no part of the line set loads on another part of the line set.

According to a further embodiment of the invention, said organizing means comprises a holding member arranged to hold at least one portion of the first tubular line element in a predetermined position in relation to a portion of the second tubular line element. By holding the two tubular line elements in a predetermined position in relation to each other, it is possible to organize these two line elements such that no part of the line elements extends across the other line element. The holding member may be arranged to perform said holding in a detachable manner. Preferably, the holding member holds detachably a portion of all tubular line elements. The tubular line elements are released from the holding member by the patient when the package is going to be used. The holding member may comprise a first elongated recess, restricted by at least one resilient jaw-shaped member, which is provided with at least one concavity for detachably holding a portion of the tubular line element. The holding member may be applied to the spiral-shaped line set such that portions of the tubular line elements located at different radial distances from the periphery of the spiral-shaped line set will be detachably held in said recess. Advantageously, the holding member is arranged to hold the two portions in a predetermined position in relation to each other, such that the tubular line elements have a substantially parallel extension in the vicinity of the holding member. The risk that said two tubular line elements extend across each other is, at least in the vicinity of the holding member, eliminated.

According to a further embodiment of the invention, the holding member is arranged to hold fixedly a second connector, which is mounted to an end of the second tubular line element. In order to position the holding member in a predetermined position in relation to the line set, it is suitable to connect the holding member to such a component of the line set. The holding member may comprise a hole extending through the holding member. The holding member may be mounted to the second connector by means of said hole, which is arranged to receive the second connector. A suitable adhesive may be applied to the contact surfaces in the hole in order to obtain a fixedly mounting of the holding member to the second connector.

According to another embodiment of the invention, the package comprises a drain bag, wherein the line set is connected to the drain bag. By hygienic reasons, it is suitable that the line set already is connected to the drain bag in the package. Furthermore, the patient does not need to connect the line set to the drain bag when the package is going to be used. The line set may be connected to the drain bag via a second connector positioned at an outer periphery of the line set. In that position, the risk is minimal that the second connector is placed in a position such it loads on a part of a tubular line element.

According to a preferred embodiment of the invention, the drain bag is foldable to form two folded parts, wherein the line set is applied in the package between the two folded parts of the drain bag. Since the drain bag is empty in the package, it is easy to fold the drain bag. Thereby, the one part of the drain bag may provide a bottom surface, which supports the line set and the other part of the drain bag may provide a surface lying on the top of the line set. The drain bag may be folded at a centerline such that the upper part and the lower part of the drain bag in a folded state have substantially the same size. The weight of the drain bag in an empty state is low. Therefore, the upper part of the drain bag exerts with an insignificant pressure on the line set. The tubular line elements may be manufactured of PVC. PVC is a plastic material having suitable properties for forming the flexible line elements of the line set. Preferably, the drain bag is manufactured of a plastic material, having higher resistance against heat than PVC. Consequently, there is no risk that any part of the drain bag is adhered to the tubular line elements during the autoclave sterilization.

According to another preferred embodiment of the invention, the holding member is arranged to engage detachably one of said folded parts of the drain bag. Thereby, the holding member prevents that an edge area of this part of the drain bag unintentionally is removed from its folded position. The holding member may comprise a second recess restricted by at least one resilient jaw-shaped member, which is provided with at least one protruding member for engaging detachably said edge area. When the edge area is inserted into the recess, the protruding member grips the edge area and holds it with a resilient force. However, it is relatively easy for a patient to remove the edge area from the recess and release the drain bag from the holding member.

According to a further embodiment of the invention, the package comprises a solution bag, wherein the line set is connected to the solution bag. By hygienic reasons, it is suitable that the line set is connected to the solution bag already in the package. Furthermore, the patient does not need to connect the line set to the solution bag when the package is going to be used. Preferably, the line set is connected to the solution bag via a first connector positioned at an outer periphery of the line set. In this position, there is a minimal risk that the first connector is positioned such that it loads on a part of a tubular line element. Advantageously, the solution bag is filled with a dialysis solution. The drain bag may be applied on top of the solution bag in the package. Thereby, the filled solution bag does not load on the line set, which is enclosed in the folded drain bag.

According to a further embodiment of the invention, the line set comprises at an end a third connector, which is connectable to a coupling member of a patient. Generally, the patient has a catheter introduced into his abdomen by operation. The catheter has an outer flexible tube provided with a connection member at an outer end, which is connectable to the connector of the line set. Preferably, the third connector is positioned at an inner periphery of the line set. The connector is here positioned in a space located in the middle of the wound line set. If this space is sufficiently large for the third connector, there is no risk that the third connector loads on a part of a tubular line element.

According to a further embodiment of the invention, the line set comprises a component in the form of at least one flow organizer, wherein said organizing means is arranged to provide a space sufficient for the flow organizer such that the flow organizer does not load on any part of the tubular line elements. Consequently, the holding member has to organize the tubular line elements such they are located at a determined distance from each other at least in the vicinity of the flow organizer. Thereby, the flow organizer obtains a space of a sufficient size between adjacent tubular line elements to be positioned into. Preferably, the package comprises a wrapping for encasing the line set and other included components of the package. In this organized state, the package is exposed to autoclave sterilization.

The object is also achieved by the method initially defined, which includes the step of organizing the line set such that no part of the line set extends across another part of the line set. Thereby, the line set obtains a shape, which substantially guarantees that no part of the line set loads on another part of the line set and especially not a tubular line element of the line set. Consequently, the risk that a part of a tubular line element is deformed during the autoclave sterilization is substantially eliminated.

### BRIEF DESCRIPTION OF THE DRAWINGS

In the following, preferred embodiments of the invention are described as examples with reference to the attached drawings, in which:
- Fig. 1: shows a line set and a drain bag during a first manufacturing step of a the package according to the invention,
- Fig. 2: shows a holding member which may be a part of the invention,
- Fig. 3: shows a cross section view of the holding member in a mounted state,
- Fig. 4: shows the line set and the drain bag during a second manufacturing step of the package,
- Fig. 5: shows the package in an organized state,
- Fig. 6: shows a second embodiment of a package in an organized state and,
- Fig. 7: shows a holding member according to a second embodiment.

### DETAILED DESCRIPTION OF PREFERRED EMBODIMENTS OF THE INVENTION

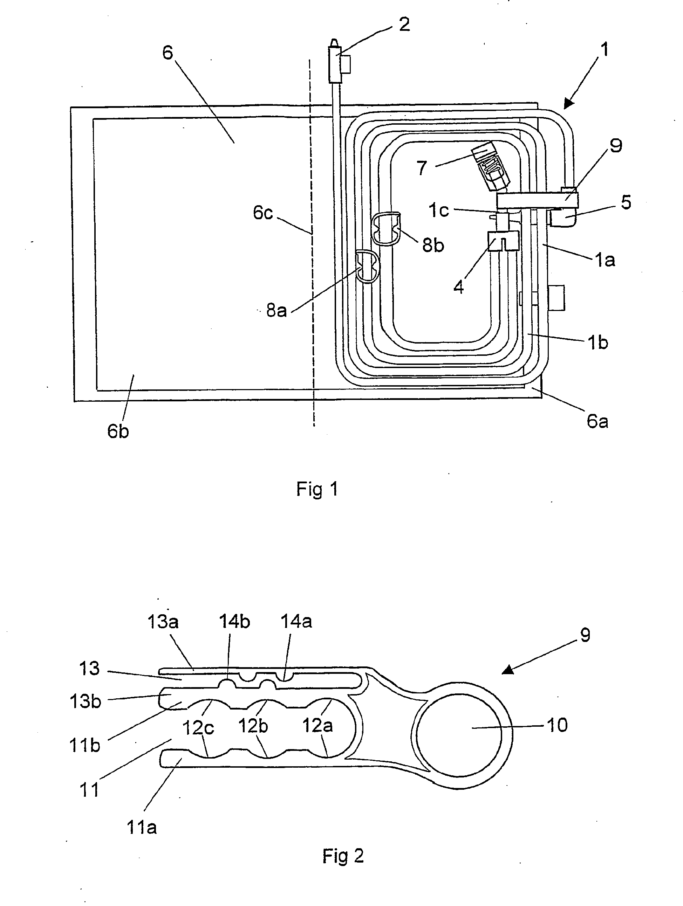

Fig. 1 shows a package including a line set 1 for use in a peritoneal dialysis treatment. The line set 1 comprises a first tubular line element 1a, a second tubular line element 1b and a third tubular line element 1c. The first tubular line element 1a has an extension between a first connector 2, which is connectable to a solution bag 3, and a junction member 4. The second tubular line element 1b has extension between a second connector 5, which is connectable to a drain bag 6, and the junction member 4. The third tubular line element 1c has an extension between a third connector 7, which is connectable to a patient, and the junction member 4. In order to control the flow in the first tubular line element 1a, a first clamp member 8a is mounted on a part of the first tubular line element 1a. In order to control the flow in the second tubular line element 1b, a second clamp member 8b is mounted on a part of the second tubular line element 1b. The tubular line elements 1a, b, c are flexible and they are manufactured of polyvinyl chloride, PVC. Alternatively, the tubular line elements, 1a, b, c will be manufactured of a material comprising polyolefin and thermoplastic elastomer.

PVC is a material having good properties for being used in the flexible line elements 1a, b, c of a line set 1. PVC has the property that it withstands an autoclaving temperature of about 120° C during a required retention time but it becomes relatively soft at this temperature. Therefore, if a part of a tubular line element 1a, b, c is loaded by another tubular line element or component in the package, there is a risk that the loaded part of the line element 1a, b, c will be permanently deformed during the autoclave sterilization. The flow of a fluid through such a deformed part of the tubular line element 1a, b, c risks to be restricted or wholly stopped. Another risk is that a loaded part of a tubular line element 1a, b, c will adhere to the loading line element or the component.

In order to avoid the above-mentioned risks, the line elements 1a, b, c are wound such that the line set 1 obtains a substantially spiral-shaped extension. Furthermore, the whole line set is organized at substantially the same level. In such a wound state, no part of the line set extends across another part of the line set. The first connector 2 to the solution bag 3 and the second connector 5 to the drain bag 6 are positioned at an outer periphery of the wound line set 1. By such an arrangement, the first connector 2 and the second connector 5 are applied in positions in which they do not load on any part of the tubular line elements 1a, b, c. The third connector 7, which is connectable to the patient, is positioned in a space at an inner periphery of the spiral shaped line set 1. In the center of the spiral shaped line set there is a space providing a sufficient size for receiving the third connector 7. In this position, the third connector 7 does not load on any part of the tubular line elements 1a, b, c.

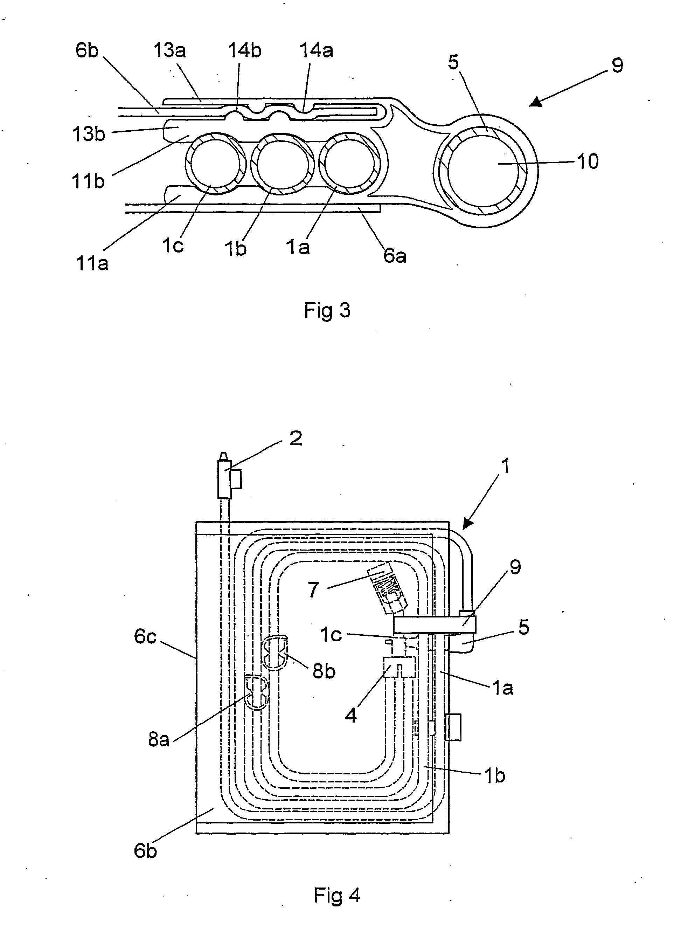

A holding member 9 is used to guarantee the organizing of the line set 1 in the package. The holding member 9 is separately shown in Fig. 2 and in a mounted state in Fig. 3. The holding member 9 has a relatively elongated shape and is provided with a hole 10 at an end portion. The hole 10 extends through the holding member 9. The hole 10 is arranged to receive and hold the second connector 5. The second connector 5 may be fixedly attached in the hole 10 of the holding member by means of an adhesive or the like. When the second connector 5 has been connected to the opening of the drain bag 6, the holding member 9 obtains a desired position in relation to the drain bag 6 in the package.

The holding member 9 is provided with a first elongated recess 11 that extends inwardly from an opposite end portion of the holding member 9 in relation to the hole 10. The holding member 9 comprises a lower jaw-shaped portion 11a and an upper jaw-shaped portion 11b. Each of the jaw-shaped portions 11a, b defines a side surface of the elongated recess 11. The lower jaw-shaped portion 11a and the upper jaw-shaped portion 11b comprise three correspondingly shaped concavities 12a, b, c arranged on opposite sides of the recess 11. The innermost concavities 12a of the jaw-shaped portions 11a, b in the recess 11 are arranged to receive a portion of the first tubular line element 1a. The intermediate concavities 12b of the jaw-shaped portions 11a, b are arranged to receive a portion of the second tubular line element 1b. The outermost concavities 12c of the jaw-shaped portions 11a, b are arranged to receive a portion of the third tubular line element 1c. In this case, the concavities 12a, b has a shape in the holding member 9 such that the attached portions of the first tubular line element 1a and the second tubular line element 1b is held in such a way that the line elements 1a, b extend in a parallel direction in the holding member 9. However, the outermost concavities 12c have a slightly inclined shape in relation to the concavities 12a, b. Therefore, the third line elements 1c obtains a curved extension in the proximity of the outermost concavities 12c. Consequently, the third connector 7 obtains an angular position in the package such that its front end portion does not load on the adjacent second tubular line element 1b.

The holding member 9 is manufactured of a plastic material having properties such that the jaw-shaped portions 11a, b obtain a suitable flexibility in a transverse direction in relation to the elongated recess 11. Thereby, it is easy to insert the tubular line elements 1a, b, c into the respective concavities 12a, b, c in the jaw-shaped portions 11a, b. The line elements 1a, b, c are, in an organized state, organized by the holding member 9 such that they are held at a predetermined distance from each other at substantially the same level. Furthermore, the holding member 9 holds the second connector 5 by the hole 10 at substantially the same level as the tubular line elements 1a, b, c. By the use of a holding member 9 having the above described design, it is possible to organize the whole line set 1 such that no part of the line set 1 extends across another part of the line set 1 in the organized state. Furthermore, the holding member 9 makes it possible to organize the line set 1 such that no parts of the tubular line elements 1a, b, c are in contact with each other at any place in the package. The holding member 9 guarantees that the tubular line elements 1a, b, c are located at a predetermined distance from each other. Thereby, a space of a required size is obtained between adjacent tubular line elements 1a, b, c in which the clamps 8a, b will be positioned. Thereby, the clamps 8a, b neither load on any part of the tubular line elements 1a, b, c. In order to facilitate further the organization of the line set 1, the tubular line elements 1a, b, c are pre-shaped to extend along a desired path, such as a spiral-like path. Consequently, they automatically try to attain said pre-shaped spiral-like shape, in which no part of the line set 1 loads on another part of the line set 1.

When the second connector 5 has been connected to the opening in the drain bag 6, the line set 1 is positioned on a first part 6a of drain bag 6, which provides a bottom surface for the line set 1. Thereby it is possible to fold the drain bag 6 at a centerline 6c, such that a second folded part 6b of the drain bag being positioned above the line set 1, see Fig. 4, and the first folded part 6a in the organized state. The holding member 9 is provided with a second elongated recess 13, which, in a mounted position, is located above the first recess 11. The holding member 9 comprises an upper jaw-shaped portion 13a and a lower jaw-shaped portion 13b. The lower jaw-shaped portion 13b is identical to the upper jaw-shaped portion 11b of the first recess 11. The upper jaw-shaped portion 13a comprises protruding members 14a and the lower jaw-shaped portion 13b comprises protruding members 14b. However, the upper protruding members 14a are displaceably arranged in relation to the lower protruding members 14b on the opposite side of the recess 13. The jaw-shaped portions 13a, b has a flexibility in a transverse direction in relation to the elongated recess 13. Thereby, it is relatively easy to introduce an edge area of the second folded part 6b of the drain bag 6 into recess 13. The jaw-shaped, portions 13a, b hold the edge area 6b with a resilient force by means of the protruding members 14a, b. In a folded, organized state, the first part 6a and the second part 6b of the drain bag 6 are in contact with the line set 1 in the package. Therefore, the drain bag 6 preferably is manufactured of another plastic material than PVC, such as a material comprising polyolefin and thermoplastic elastomer. The drain bag 6 is manufactured of a plastic material having a higher resistance against heat than PVC. Thereby, the plastic material of the drain bag 6 does not soften during the autoclaving temperature. Consequently, there is no risk that the tubular line elements 1a, b, c adhere to the drain bag 6. The drain bag 6 may be provided with an embossed pattern.

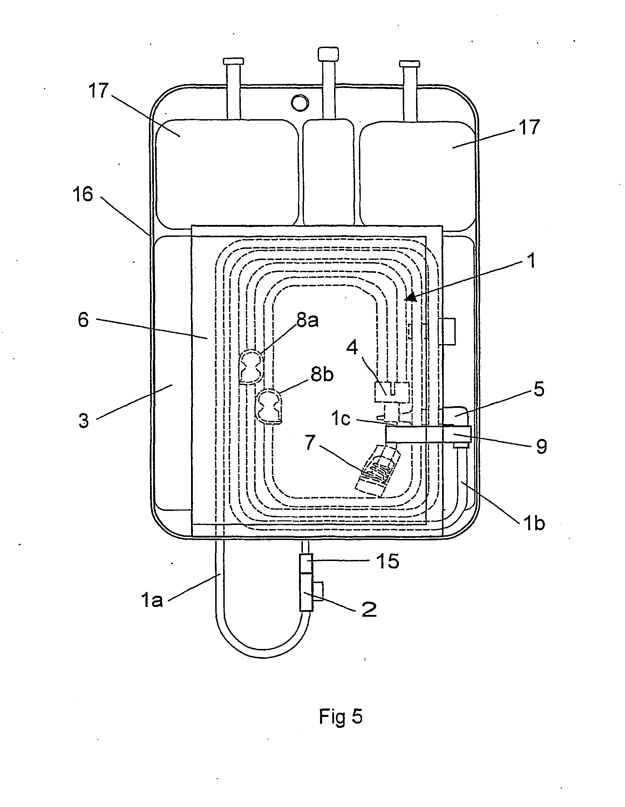

Fig. 5 shows the package in the organized state to be exposed to autoclave sterilization. The line set 1 and the drain bag 6, which is folded around the line set 1, are in the package applied on the solution bag 3. The solution bag 3 is filled with a dialysis solution. The first connector 2 is connected to a connection member 15 of the solution bag 3. A number of containers 17 are formed in the solution bag 3. Finally, a wrapping 16 has been wrapped around the package for completely encasing the line set 1, the bags 3, 6 and the other components of the package.

When the package is to be used for a peritoneal dialysis treatment, the patient initially removes the wrapping 16. After that the edge portion of the second part 6b is removed from the detachable attachment in the second recess 13 of the holding member 9. The second part 6b of the drain bag is unfolded from its position above the line set 1 such that the line set 1 becomes uncovered. The patient grips the holding member 9 and separates the line elements 1a, b, c from the detachable attachment in the first recess 11 of the holding member 9. The peritoneal dialysis set is now ready to be used. The connector 7 connects to a catheter of the patient. The patient sets the clamp 8a in a closed position and the clamp 8b in an open position such that used fluid contained in the abdominal cavity is drained through the third tubular line element 1c and the tubular second line element 1b to the drain bag 6. Thereafter, the patient sets the clamp 8a in an open position and the clamp 8b in a closed position. Fresh dialysis solution flows now from the solution bag 3, via the first tubular line element 1a and the third tubular line element 1c, to the abdominal cavity of the patient.

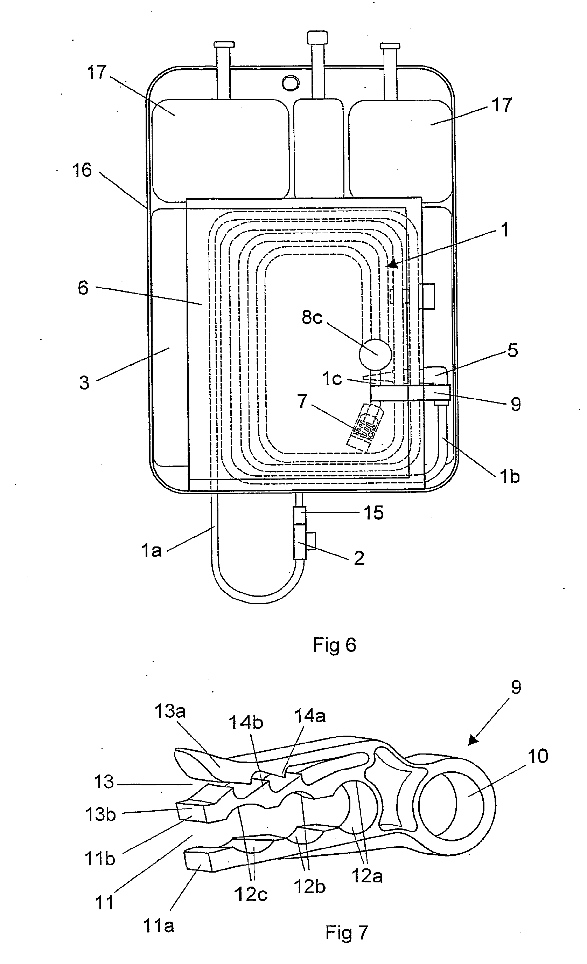

Fig. 6 shows an alternative package in the organized state, which is to be exposed to autoclave sterilization. In this case, the package comprises a flow organizer in the form of a valve 8c for controlling the flow in the first tubular line element 1a and in the second tubular line element 1b. The valve 8c is arranged in a connection between the first tubular line element 1a and the second tubular line element 1b. Consequently, a separate junction member 4 does not need to be used. In the organized package, the valve 8c is positioned at an inner periphery of the spiral-shaped line set 1. Otherwise, the package has a correspondence design as the package shown in Fig. 5.

Fig. 7 shows an alternative embodiment of the holding member 9. It is here visible that the outermost concavity 12c has a slightly inclined shape in relation to the concavities 12a, b. The inclined outermost concavity 12c guarantees that the third line element 1c obtains a curved extension in the proximity of the outermost concavity 12c such that the third connector 7 obtains an angular position in the package. Thereby, the third connector 7 does not load on the adjacent second tubular line element 1b. The upper jaw-shaped portion 13a has here a free end, which is inclined such that the inlet to the second recess 13 obtains a larger size than the remaining part of the second recess 13. Thereby, it is simple to introduce the edge part of the drain bag 6 into the second recess 13.

The present invention is not limited to the embodiments disclosed, but may be varied and modified within the scope of the claims.

## Claims

1. A package for use in a peritoneal dialysis treatment, wherein the package includes a line set (1), which comprises a first tubular line element (1a), a second tubular line element (1b) and at least one component (2, 5, 7, 4, 8a, 8b, 8c) connected to the tubular line elements (1a, 1b), **characterized in that** the package comprises organizing means (9) arranged to organize the line set (1) such that in the package no part of the line set (1) extends across another part of the line set (1).

2. A package according to claim 1, **characterized in that** the organizing means (9) is arranged to organize the whole line set (1) at substantially the same level.

3. A package according to claim 1 or 2, **characterized in that** the organizing means is arranged to organize the line set (1) such that no part of the tubular line elements (1a, 1b, 1c) is in contact with another part of the tubular line elements (1a, 1b, 1c).

4. A package according to any one of the preceding claims, **characterized in that** that the organizing means (9) is arranged to organize the line set (1) in a spiral-shaped state.

5. A package according to any one of the preceding claims, **characterized in that** at least one tubular line element (1a, 1b, 1c) is pre-shaped to extend along a desired path.

6. A package according to any one of the preceding claims, **characterized in that** said organizing means comprises a holding member (9) arranged to hold at least one portion of the first tubular line element (1a) in a predetermined position in relation to a portion of the second tubular line element (1b).

7. A package according to claim 6, **characterized in that** the holding member (9) is arranged to perform said holding in a detachable manner.

8. A package according to claim 7, **characterized in that** the holding member (9) comprises a first elongated recess (11), restricted by at least one resilient jaw-shaped member (11a, 11b), which is provided with at least one concavity (12a, 12b, 12c) for holding detachably said portion of the tubular line element (1a, 1b, 1c).

9. A package according to any one of the claims 6 to 8, **characterized in that** the holding member (9) is arranged to hold the two portions, which are positioned in a predetermined position in relation two each other, such that the tubular line elements have a substantially parallel extension in the vicinity of the holding member (9).

10. A package according to any one of the preceding claims 7 to 10, **characterized in that** the holding member (9) is arranged to hold fixedly a second connector (5), which is mounted to an end of the second tubular line element (1b).

11. A package according to claim 10, **characterized in that** the holding member (9), comprises a hole (10) extending through the holding member (9) for receiving said connector (5).

12. A package according to any one of the preceding claims, **characterized in that** the package comprises a drain bag (6), wherein the line set (1) is connected to the drain bag (6).

13. A package according to any one of the preceding claims, **characterized in that** the tubular line elements (1a, 1b, 1c) are manufactured of PVC.

14. A package according to claim 12 and 13, **characterized in that** the drain bag (3) is manufactured of a plastic material having higher resistance against heat than PVC.

15. A package according to any one of the claims 12 to 14, **characterized in that** the drain bag (3) is foldable to form two folded parts (6a, 6b), wherein the line set (1) is positioned in the package between the two folded parts (6a, 6b) of the drain bag (6).

16. A package according to claim 15, **characterized in that** the holding member (9) is arranged to detachably engage one of said folded parts (6a, 6b) of the drain bag (6).

17. A package according to claim 16, **characterized in that** the holding member (9) comprises a second recess (13) restricted by at least one resilient jaw-shaped member (13a, 13b), which is provided with at least one protruding member (14) for engaging detachably said edge area.

18. A package according to any one of the claims 12 to 17, **characterized in that** the line set (1) is connected to the drain bag (6) via a first connector (2) positioned at an outer periphery of the line set (1).

19. A package according to any one of the preceding claims, **characterized in that** the package comprises a solution bag (3), wherein the line set (1) is connected to the solution bag (3).

20. A package according to claim 15 and 19, **characterized in that** the drain bag (6) is applied on the solution bag (3).

21. A package according to claim 18 and 19, **characterized in** the line set (1) is connected to the solution bag (3) via said first connector (2) positioned at an outer periphery of the line set (1).

22. A package according to any one of the claims 19 to 21, **characterized in that** the solution bag (3) is filled with a dialysis solution.

23. A package according to any one of the preceding claims, **characterized in that** the line set (1) comprises a third connector (7), which is connectable to a patient connector.

24. A package according to claim 23, **characterized in that** the third connector (7) is arranged in a space at an inner periphery of the line set (1).

25. A package according to any one of the preceding claims, **characterized in that** line set comprises a component in the form of at least one flow organizer (8a, 8b, 8c), wherein said organizing means (9) is arranged to provide a space sufficient for the flow organizer (8a, 8b, 8c) such that the flow organizer (8a, 8b, 8c) does not load on any part of the tubular line elements (1a, 1b, 1c).

26. A package according to any one of the preceding claims, **characterized in that** package comprises a wrapping (16) for encasing the line set (1) and other included parts (3, 6) of the package.

27. A method for manufacturing of a package for use in a peritoneal dialysis treatment, wherein the package includes a line set (1), which comprises a first tubular line element (1a), a second tubular line element (1b) and at least one component (2, 5, 7, 4, 8a, 8b, 8c) connected to the tubular line elements (1a, 1b, 1c), **characterized by** the step of organizing the line set (1) such that in the package no part of the line set (1) extends across another part of the line set (1).

28. A method according to claim 27, **characterized by** the step of organizing the whole line set (1) at substantially the same level.

29. A method according to claim 27 or 28, **characterized by** the step of organizing the line set (1) such that no part of the tubular line elements (1a, 1b, 1c) is in contact with another part of the tubular line elements.

30. A method according to any one of the claims 27 to 29 **characterized by** the step of organizing the line set (1) in a spiral-shaped state.

31. A method according to any one of the claims 27 to 30, **characterized by** the step of organizing the line set (1) by means of a holding member (9), which holds at least one portion of the first tubular line element (1a) in a predetermined position in relation to a portion of the second tubular line element (1b).

32. A method according to any one of the claims 27 to 31, wherein the package comprises a drain bag (6), **characterized by** the steps of folding the drain bag (3) such it forms two folded parts (6a, 6b) and applying the line set between the two folded parts (6a, 6b) of the drain bag (6).

33. A method according to claim 32, wherein the package comprises a solution bag (3), **characterized by** the step of applying the drain bag (6) on the solution bag (3).

34. A method according to any one of the claims 27 to 33, **characterized by** the step of providing the package with a wrapping.

35. A method according to any one of the claims 27 to 34, **characterized by** the step of exposing the package for autoclave sterilization.

## Patentansprüche

1. Packung zur Verwendung bei einer Peritonealdialysebehandlung, wobei die Packung einen Leitungssatz (1) aufweist, der ein erstes Rohrleitungselement (1a), ein zweites Rohrleitungselement (1b) und zumindest eine Komponente (2, 5, 7, 4, 8a, 8b, 8c) umfasst, die mit den Rohrleitungselementen (1a, 1b) verbunden ist, **dadurch gekennzeichnet, dass** die Packung ein Organisierungsmittel (9) umfasst, das derart angeordnet ist, um den Leitungssatz (1) so zu organisieren, dass sich in der Packung kein Teil des Leitungssatzes (1) über ein anderes Teil des Leitungssatzes (1) erstreckt.

2. Packung nach Anspruch 1, **dadurch gekennzeichnet, dass** das Organisierungsmittel (9) derart angeordnet ist, um den gesamten. Leitungssatz (1) auf im Wesentlichen demselben Niveau zu organisieren.

3. Packung nach Anspruch 1 oder 2, **dadurch gekennzeichnet, dass** das Organisierungsmittel derart angeordnet ist, um den Leitungssatz (1) so zu organisieren, dass kein Teil der Rohrleitungselemente (1a, 1b, 1c) mit einem anderen Teil der Rohrleitungselemente (1a, 1b, 1c) in Kontakt steht.

4. Packung nach einem der vorhergehenden Ansprüche, **dadurch gekennzeichnet, dass** das Organisierungsmittel (9) derart angeordnet ist, um den Leitungssatz (1) in einem spiralförmigen Zustand zu organisieren.

5. Packung nach einem der vorhergehenden Ansprüche, **dadurch gekennzeichnet, dass** zumindest ein Rohrleitungselement (1a, 1b, 1c) vorgeformt ist, um entlang eines gewünschten Pfades zu verlaufen.

6. Packung nach einem der vorhergehenden Ansprüche, **dadurch gekennzeichnet, dass** das Organisierungsmittel ein Halteelement (9) umfasst, das derart angeordnet ist, um zumindest einen Abschnitt des ersten Rohrleitungselements (1a) in einer vorbestimmten Position in Bezug auf einen Abschnitt des zweiten Rohrleitungselements (1b) zu halten.

7. Packung nach Anspruch 6, **dadurch gekennzeichnet, dass** das Halteelement (9) derart angeordnet ist, um das Halten in einer lösbaren Art und Weise auszuführen.

8. Packung nach Anspruch 7, **dadurch gekennzeichnet, dass** das Halteelement (9) eine erste längliche Ausnehmung (11) umfasst, die durch zumindest ein nachgiebiges klauenförmiges Element (11a, 11b) begrenzt ist, das mit zumindest einer Höhlung (12a, 12b, 12c) versehen ist, um den Abschnitt des Rohrleitungselements (1a, 1b, 1c) lösbar zu halten.

9. Packung nach einem der Ansprüche 6 bis 8, **dadurch gekennzeichnet, dass** das Halteelement (9) derart angeordnet ist, um die beiden Abschnitte, die in einer vorbestimmten Position in Bezug zueinander positioniert sind, so zu halten, dass die Rohrleitungselemente eine im Wesentlichen parallele Erstreckung in der Nähe des Halteelements (9) besitzen.

10. Packung nach einem der vorhergehenden Ansprüche 7 bis 10, **dadurch gekennzeichnet, dass** das Halteelement (9) derart angeordnet ist, um einen zweiten Verbinder (5) fixiert zu halten, der an einem Ende des zweiten Rohrleitungselements (1b) angebracht ist.

11. Packung nach Anspruch 10, **dadurch gekennzeichnet, dass** das Halteelement (9) ein Loch (10) umfasst, das zur Aufnahme des Verbinders (5) durch das Halteelement (9) verläuft.

12. Packung nach einem der vorhergehenden Ansprüche, **dadurch gekennzeichnet, dass** die Packung einen Ablassbeutel (6) umfasst, wobei der Leitungssatz (1) mit dem Ablassbeutel (6) verbunden ist.

13. Packung nach einem der vorhergehenden Ansprüche, **dadurch gekennzeichnet, dass** die Rohrleitungselemente (1a, 1b, 1c) aus PVC hergestellt sind.

14. Packung nach Anspruch 12 oder 13, **dadurch gekennzeichnet, dass** der Ablassbeutel (3) aus einem Kunststoffmaterial hergestellt ist, das eine höhere Beständigkeit gegenüber Wärme als PVC besitzt.

15. Packung nach einem der Ansprüche 12 bis 14, **dadurch gekennzeichnet, dass** der Ablassbeutel (3) faltbar ist, um zwei gefaltete Teile (6a, 6b) zu bilden, wobei der Leitungssatz (1) in der Packung zwischen den beiden gefalteten Teilen (6a, 6b) des Ablassbeutels (6) positioniert ist.

16. Packung nach Anspruch 15, **dadurch gekennzeichnet, dass** das Halteelement (9) derart angeordnet ist, um eines der gefalteten Teile (6a, 6b) des Ablassbeutels (6) lösbar in Eingriff zu bringen.

17. Packung nach Anspruch 16, **dadurch gekennzeichnet, dass** das Halteelement (9) eine zweite Ausnehmung (13) umfasst, die durch zumindest ein nachgiebiges klauenförmiges Element (13a, 13b) begrenzt ist, das mit zumindest einem vorstehenden Element (14) zum lösbaren Eingriff mit dem Randbereich versehen ist.

18. Packung nach einem der Ansprüche 12 bis 17, **dadurch gekennzeichnet, dass** der Leitungssatz (1) mit dem Ablassbeutel (6) über einen ersten Verbinder (2) verbunden ist, der an einem Außenumfang des Leitungssatzes (1) positioniert ist.

19. Packung nach einem der vorhergehenden Ansprüche, **dadurch gekennzeichnet, dass** die Packung einen Lösungsbeutel (3) umfasst, wobei der Leitungssatz (1) mit dem Lösungsbeutel (3) verbunden ist.

20. Packung nach einem der Ansprüche 15 und 19, **dadurch gekennzeichnet, dass** der Ablassbeutel (6) an dem Lösungsbeutel (3) angebracht ist.

21. Packung nach Anspruch 18 oder 19, **dadurch gekennzeichnet, dass** der Leitungssatz (1) mit dem Lösungsbeutel (3) über den ersten Verbinder (2) verbunden ist, der an einem Außenumfang des Leitungssatzes (1) positioniert ist.

22. Packung nach einem der Ansprüche 19 bis 21, **dadurch gekennzeichnet, dass** der Lösungsbeutel (3) mit einer Dialyselösung gefüllt ist.

23. Packung nach einem der vorhergehenden Ansprüche, **dadurch gekennzeichnet, dass** der Leitungssatz (1) einen dritten Verbinder (7) umfasst, der mit einem Patientenverbinder verbindbar ist.

24. Packung nach Anspruch 23, **dadurch gekennzeichnet, dass** der dritte Verbinder (7) in einem Raum an einem Innenumfang des Leitungssatzes (1) angeordnet ist.

25. Packung nach einem der vorhergehenden Ansprüche, **dadurch gekennzeichnet, dass** der Leitungssatz eine Komponente in der Form zumindest einer Strömungsorganisierungseinrichtung (8a, 8b, 8c) umfasst, wobei das Organisierungsmittel (9) derart angeordnet ist, um einen Raum bereitzustellen, der für die Strömungsorganisierungseinrichtung (8a, 8b, 8c) ausreichend ist, so dass die Strömungsorganisierungseinrichtung (8a, 8b, 8c) nicht auf irgendeinem Teil der Rohrleitungselemente (1a, 1b, 1c) lastet.

26. Packung nach einem der vorhergehenden Ansprüche, **dadurch gekennzeichnet, dass** die Packung eine Umwicklung (16) umfasst, um den Leitungssatz (1) und andere enthaltene Teile (3, 6) der Packung einzuschließen.

27. Verfahren zur Herstellung einer Packung zur Verwendung bei einer Peritonealdialysebehandlung, wobei die Packung einen Leitungssatz (1) aufweist, der ein erstes Rohrleitungselement (1a), ein zweites Rohrleitungselement (1b) und zumindest eine Komponente (2, 5, 7, 4, 8a, 8b, 8c) umfasst, die mit den Rohrleitungselementen (1a, 1b, 1c) verbunden ist, **gekennzeichnet durch** den Schritt, dass der Leitungssatz (1) so organisiert wird, dass sich in der Packung kein Teil des Leitungssatzes (1) über ein anderes Teil des Leitungssatzes (1) erstreckt.

28. Verfahren nach Anspruch 27, **gekennzeichnet durch** den Schritt, dass der gesamte Leitungssatz (1) auf im Wesentlichen demselben Niveau organisiert wird.

29. Verfahren nach Anspruch 27 oder 28, **gekennzeichnet durch** den Schritt, dass der Leitungssatz (1) so organisiert wird, dass kein Teil der Rohrleitungselemente (1a, 1b, 1c) in Kontakt mit einem anderen Teil der Rohrleitungselemente steht.

30. Verfahren nach einem der Ansprüche 27 bis 29, **gekennzeichnet durch** den Schritt, dass der Leitungssatz (1) in einem spiralförmigen Zustand organisiert wird.

31. Verfahren nach einem der Ansprüche 27 bis 30, **gekennzeichnet durch** den Schritt, dass der Leitungssatz (1) mittels eines Halteelements (9) organisiert wird, der zumindest einen Abschnitt des ersten Rohrleitungselements (1a) in einer vorbestimmten Position in Bezug auf einen Abschnitt des zweiten Rohrleitungselements (1b) hält.

32. Verfahren nach einem der Ansprüche 27 bis 31, wobei die Packung einen Ablassbeutel (6) umfasst, **gekennzeichnet durch** die Schritte, dass der Ablassbeutel (3) so gefaltet wird, dass er zwei gefaltete Teile (6a, 6b) bildet, und der Leitungssatz zwischen den beiden gefalteten Teilen (6a, 6b) des Ablassbeutels (6) angebracht wird.

33. Verfahren nach Anspruch 32, wobei die Packung einen Lösungsbeutel (3) umfasst, **gekennzeichnet durch** den Schritt, dass der Ablassbeutel (6) an dem Lösungsbeutel (3) angebracht wird.

34. Verfahren nach einem der Ansprüche 27 bis 33, **gekennzeichnet durch** den Schritt, dass die Packung mit einer Umwicklung versehen wird.

35. Verfahren nach einem der Ansprüche 27 bis 34, **gekennzeichnet durch** den Schritt, dass die Packung einer Autoklavensterilisierung ausgesetzt wird.

## Revendications

1. Conditionnement destiné à être utilisé dans un traitement de dialyse péritonéale, dans lequel le conditionnement comprend un ensemble de lignes (1), qui comporte un premier élément de ligne tubulaire (1a), un deuxième élément de ligne tubulaire (1b) et au moins un composant (2, 5, 7, 4, 8a, 8b, 8c) connecté aux éléments de ligne tubulaires (1a, 1b), **caractérisé en ce que** le conditionnement comporte des moyens d'organisation (9) agencés pour organiser l'ensemble de lignes (1) de façon que, dans le conditionnement, aucune partie de l'ensemble de lignes (1) ne s'étend sur une autre partie de l'ensemble de lignes (1).

2. Conditionnement selon la revendication une, **caractérisé en ce que** les moyens d'organisation (9) sont agencés pour organiser la totalité de l'ensemble de lignes (1) sensiblement à un même niveau.

3. Conditionnement selon l'une des revendications 1 ou 2, **caractérisé en ce que** les moyens d'organisation sont agencés pour organiser l'ensemble de tubes (1) de manière qu'aucune partie des éléments de ligne tubulaires (1a, 1b, 1c) n'est en contact avec une autre partie des éléments de ligne tubulaires (1a, 1b, 1c).

4. Conditionnement selon l'une quelconque des revendications précédentes, **caractérisé en ce que** les moyens d'organisation (9) sont agencés pour organiser l'ensemble de lignes (1) dans un état en forme de spirale.

5. Conditionnement selon l'une quelconque des revendications précédentes, **caractérisé en ce que** au moins un élément de ligne tubulaire (1a, 1b, 1c) est préformé pour s'étendre le long d'un trajet désiré.

6. Conditionnement selon l'une quelconque des revendications précédentes, **caractérisé en ce que** lesdits moyens d'organisation comportent un élément de support (9) agencé pour supporter au moins un tronçon du premier élément de ligne tubulaire (1a) dans une position prédéterminée par rapport à un tronçon du second élément de ligne tubulaire (1 b.).

7. Conditionnement selon la revendication 6, **caractérisé en ce que** l'élément de support (9) est agencé pour réaliser ledit support de manière détachable.

8. Conditionnement selon la revendication 7, **caractérisé en ce que** l'élément de support (9) comporte un premier évidement allongé (11), restreint par au moins un élément en forme de mâchoire élastique (11a, 11b), qui est muni d'au moins une concavité (12a, 12b, 12c) pour supporter de manière détachable ladite portion de l'élément de ligne tubulaire (1a, 1b, 1c).

9. Conditionnement selon l'une quelconque des revendications 6 à 8, **caractérisé en ce que** l'élément de support (9) est agencé pour supporter les deux tronçons, qui sont positionnées dans une position prédéterminée l'un par rapport à l'autre, de telle façon que les éléments de ligne tubulaires ont un prolongement sensiblement parallèle dans le voisinage de l'élément de support (9).

10. Conditionnement selon l'une quelconque des revendications précédentes 7 à 10, **caractérisé en ce que** l'élément de support (9) est agencé pour supporter de manière fixe un deuxième connecteur (5), qui est monté à une extrémité du deuxième élément de ligne tubulaire (1 b).

11. Conditionnement selon la revendication 10, **caractérisé en ce que** l'élément de support (9), comporte un trou (10) s'étendant à travers l'élément de support (9) pour recevoir ledit connecteur (5).

12. Conditionnement selon l'une quelconque des revendications précédentes, **caractérisé en ce que** le conditionnement comporte un sac de drainage (6), dans lequel l'ensemble de lignes (1) est connecté au sac de drainage (6).

13. Conditionnement selon l'une quelconque des revendications précédentes, **caractérisé en ce que** les éléments de ligne tubulaire (1a, 1b, 1c) sont fabriqués en PVC.

14. Conditionnement selon les revendications 12 et 13, **caractérisé en ce que** le sac de drainage (3) est fabriqué en matière plastique ayant une résistance à la chaleur plus élevée que celle du PVC.

15. Conditionnement selon l'une quelconque des revendications 12 à 14, **caractérisé en ce que** le sac de drainage (3) est pliable pour former deux parties pliées (6a, 6b) dans lequel l'ensemble de lignes (1) est positionné dans le conditionnement entre les deux parties pliées (6a, 6b) du sac de drainage (6).

16. Conditionnement selon la revendication 15, **caractérisé en ce que** l'élément de support (9) est agencé pour coopérer de manière détachable avec une desdites parties pliées (6a, 6b) du sac de drainage (6).

17. Conditionnement selon la revendication 16, **caractérisé en ce que** l'élément de support (9) comporte un deuxième évidement (13) restreint par au moins un élément en forme de mâchoire élastique (13a, 13b) qui est muni d'au moins un élément en saillie (14) pour coopérer de manière détachable avec ladite zone de bord.

18. Conditionnement selon l'une quelconque des revendications 12 à 17, **caractérisé en ce que** l'ensemble de lignes (1) est connecté au sac de drainage (6) à travers un premier connecteur (2) positionné à une périphérie externe de l'ensemble de lignes (1).

19. Conditionnement selon l'une quelconque des revendications précédentes, **caractérisé en ce que** le conditionnement comporte un sac de solution (3), dans lequel l'ensemble de lignes (1) est connecté au sac de solution (3).

20. Conditionnement selon les revendications 15 et 19, **caractérisé en ce que** le sac de drainage (6) est appliqué sur le sac de solution (3).

21. Conditionnement selon les revendications 18 et 19, **caractérisé en ce que** l'ensemble de lignes (1) est connecté au sac de solution (3) à travers le dit premier connecteur (2) positionné à une périphérie externe de l'ensemble de lignes (1).

22. Conditionnement selon l'une quelconque des revendications 19 à 21, **caractérisé en ce que** le sac de solution (3) est rempli avec une solution de dialyse.

23. Conditionnement selon l'une quelconque des revendications précédentes, **caractérisé en ce que** l'ensemble de lignes (1) comporte un troisième connecteur (7), qui peut être connecté à un connecteur de patient.

24. Conditionnement selon la revendication 23, **caractérisé en ce que** le troisième connecteur (7) est agencé dans un espace à une périphérie interne de l'ensemble de lignes (1).

25. Conditionnement selon l'une quelconque des revendications précédentes, **caractérisé en ce que** l'ensemble de lignes comporte un composant sous la forme d'au moins un organiseur d'écoulements (8a, 8b, 8c), dans lequel lesdits moyens d'organisation (9) sont agencées pour fournir un espace suffisant pour l'organiseur d'écoulements (8a, 8b, 8c) de manière que l'organiseur d'écoulements (8a, 8b, 8c) n'applique de charge sur aucune partie des éléments de ligne tubulaires (1a, 1b, 1c).

26. Conditionnement selon l'une quelconque des revendications précédentes, **caractérisé en ce que** le conditionnement comporte un emballage (16) pour loger l'ensemble de lignes (1) et les autres parties (3, 6) incluses dans le conditionnement.

27. Procédé pour fabriquer un conditionnement destiné à être utilisé dans un traitement de dialyse péritonéale, dans lequel le conditionnement comprend un ensemble de lignes (1), qui comporte un premier élément de ligne tubulaire (1a), un deuxième élément de ligne tubulaire (1b) et au moins un composant (2, 5, 7, 4, 8a, 8b, 8c) connecté aux éléments de ligne tubulaires (1a, 1b), **caractérisé par** une étape consistant à organiser l'ensemble de lignes (1) de telle manière que, dans le conditionnement, aucune partie de l'ensemble de lignes ne s'étend sur une autre partie de l'ensemble de lignes (1).

28. Procédé selon la revendication 27, **caractérisé par** une étape consistant à organiser la totalité de l'ensemble de lignes (1) sensiblement à un même niveau.

29. Procédé selon l'une des revendications 27 ou 28, **caractérisé par** une étape consistant à organiser l'ensemble de lignes (1) de telle manière qu'aucune partie des éléments de ligne tubulaires (1a, 1b, 1c) n'est en contact avec une autre partie des éléments de ligne tubulaires (1a, 1b, 1c).

30. Procédé selon l'une quelconque des revendications 27 à 29, **caractérisé par** une étape consistant à organiser l'ensemble de lignes (1) dans un état en forme de spirale.

31. Procédé selon l'une quelconque des revendications 27 à 30, **caractérisé par** une étape consistant à organiser l'ensemble de lignes (1) au moyen d'un élément de support (9) qui supporte au moins un tronçon du premier élément de ligne tubulaire (1a) dans une position prédéterminée par rapport à un tronçon du second élément de ligne tubulaire (1 b.).

32. Procédé selon l'une quelconque des revendications 27 à 31, dans lequel le conditionnement comporte un sac de drainage (6) **caractérisé par** les étapes consistant à plier le sac de drainage (3) de telle manière qu'il forme deux parties pliées (6a, 6b) et à appliquer l'ensemble de lignes entre les deux parties pliées (6a, 6b) du sac de drainage.

33. Procédé selon la revendication 32, dans lequel le conditionnement comporte un sac de solution (3), **caractérisé par** l'étape consistant à appliquer le sac de drainage (6) sur le sac de solution (3).

34. Procédé selon l'une quelconque des revendications 27 à 33, **caractérisé par** l'étape consistant à munir le conditionnement d'un emballage.

35. Procédé selon l'une quelconque des revendications 27 à 34, **caractérisé par** l'étape consistant à exposer le conditionnement à une stérilisation en autoclave.
